(19) Europäisches Patentamt
European Patent Office
Office européen des brevets

(11) EP 0 638 539 B1

(12) EUROPÄISCHE PATENTSCHRIFT

(45) Veröffentlichungstag und Bekanntmachung des Hinweises auf die Patenterteilung:
**03.11.1999 Patentblatt 1999/44**

(51) Int. Cl.[6]: **C07C 53/48**, C07C 53/50, C07C 51/58

(21) Anmeldenummer: **94112316.8**

(22) Anmeldetag: **06.08.1994**

# (54) Verfahren zur Herstellung von Poly- und Perfluorcarbonsäurechloriden

Process for preparing poly- and perfluorocarboxylic acid chlorides

Procédé pour préparer des chlorures d'acides carboxyliques poly- et perfluorés

(84) Benannte Vertragsstaaten:
**AT BE CH DE DK ES FR GB GR IE IT LI LU NL PT SE**

(30) Priorität: **13.08.1993 DE 4327195**
**14.12.1993 DE 4342601**

(43) Veröffentlichungstag der Anmeldung:
**15.02.1995 Patentblatt 1995/07**

(73) Patentinhaber:
**Solvay Fluor und Derivate GmbH**
**D-30173 Hannover (DE)**

(72) Erfinder:
- **Braun, Dr. Max**
  **D-30938 Burgwedel (DE)**
- **Rudolph, Dr. Werner**
  **D-30559 Hannover (DE)**
- **Eichholz, Kerstin**
  **D-30855 Langenhagen (DE)**

(74) Vertreter: **Lauer, Dieter, Dr.**
**Solvay Pharmaceuticals GmbH,**
**Hans-Böckler-Allee 20**
**30173 Hannover (DE)**

(56) Entgegenhaltungen:
**DE-B- 1 254 616** **DE-C- 2 418 676**
**US-A- 3 151 051**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7/3.6

## Beschreibung

Die Erfindung bezieht sich auf ein verfahren zur Herstellung von Polyfluorchlor- und Perfluorcarbonsäurechloriden, insbesondere von Trifluoracetylchlorid und Chlordifluoracetylchlorid.

Polyfluorchlor- und Perfluorcarbonsäurechloride sind Bausteine in der chemischen Synthese, beispielsweise für die Tensidherstellung, Pharmazeutika und Agrochemikalien.

Chlordifluoracetylchlorid ist ein Zwischenprodukt, das in mannigfaltiger Weise in der chemischen Synthese eingesetzt werden kann. Beispielsweise kann es bei der Herstellung von Farbstoffen eingesetzt werden. Alkyl- und Arylhalogenide können durch ein Derivat von Chlordifluoracetylchlorid, nämlich durch den Methylester, in Anwesenheit von Kaliumfluorid und Kupferjodid trifluormethyliert werden. Bislang wurde Chlordifluoracetylchlorid durch Solvolyse von $CF_2ClCCl_3$ mit Oleum oder $SO_3$ in Anwesenheit von Quecksilberverbindungen und Chlorschwefeloxiden hergestellt, wie in der DE-OS 19 17 630 beschrieben. Der erwähnte Methylester ist auch Vorprodukt für die Herstellung von Difluorcarben, s. G. A. Wheaton, D. J. Donald in J. Fluorine Chem. 8 (1976), Seiten 97 bis 100. Difluorcarben wird bei der Insektizid-Herstellung eingesetzt, siehe EP-A 198 791 (US-A 4 701 563). Die Herstellung von Difluorcarben aus $PhHgCF_3$ und anderen Verbindungen dieser Art ist aus Umweltgesichtspunkten problematisch.

Trifluoracetylchlorid ist ebenfalls ein wichtiges Zwischenprodukt in der chemischen Synthese. Die Umsetzung mit Trifluorethanol führt zum entsprechenden Ester, der durch Hydrierung in zwei Moleküle Trifluorethanol gespalten werden kann. Trifluorethanol ist ein Lösungsmittel, das auch in Trocknungs- und Reinigungsverfahren eingesetzt werden kann.

Die Herstellung von Difluorchloracetylchlorid aus 1.1-Difluor-1.2.2-trichlorethan und Sauerstoff unter photochemischer Bestrahlung durch Quarzbrenner wird in der DE-AS 1 069 137 beschrieben; als Zusatz wird Chlor verwendet, obwohl es dabei zur Bildung unerwünschter Nebenprodukte kommt.

Die DE-A 24 18 676 offenbart ein Verfahren zur Herstellung von Trifluoracetylchlorid durch photochemische Oxidation von wasserfreiem 1,1-Dichlor-2,2,2-trifluorethan (R 123) in Gegenwart von Sauerstoff bei einer Temperatur von bis zu 250 °C in der Gasphase. Bezüglich des Druckes wird erläutert, daß er im allgemeinen über 3,5 bar liegen soll, jedoch soll eine Flüssigphasenbildung vermieden werden. Chlor beschleunigt die Reaktionsgeschwindigkeit, führt jedoch zur Bildung von Nebenprodukten.

Aufgabe der vorliegenden Erfindung ist es, ein Verfahren zur Herstellung von Polyfluorchlor- und Perfluorcarbonsäurechloriden, insbesondere von Trifluoracetylchlorid und Chlordifluoracetylchlorid anzugeben, welches ohne Zusatz von Quecksilberverbindungen durchgeführt werden kann und hohe Ausbeuten bei hoher Reaktionsgeschwindigkeit und hoher Selektivität ergibt. Diese Aufgabe wird durch das Verfahren der vorliegenden Erfindung gelöst.

Erfindungsgemäß stellt man Verbindungen der allgemeinen Formel RCFXC(O)Cl, worin X für Fluor oder Chlor steht und R für Fluor oder einen perfluorierten C1- C10-Alkylrest her durch Umsetzung von $RCFXCHCl_2$, worin R und X die obengenannte Bedeutung besitzen, mit Sauerstoff in der Gasphase unter aktivierender Bestrahlung, mit der Maßgabe, daß man die Umsetzung kontinuierlich ohne Zusatz von elementarem Chlor drucklos durchführt. R bedeutet vorzugsweise C1-C3-Alkyl (perfluoriert).

"Drucklos" bedeutet im Rahmen der vorliegenden Erfindung, daß auf die Reaktionsmischung außer dem Umgebungsdruck, dem Förderdruck des Sauerstoffgases (bzw. des sauerstoffhaltigen Gases) und dem sich gegebenenfalls ausbildenden Druck durch bei der Reaktion entstehendes HCl-Gas kein zusätzlicher Druck einwirkt.

Mit dem erfindungsgemäßen Verfahren kann beispielsweise 1.1.1.2.2.-Pentafluor-3.3.-dichlorpropan in Perfluorpropionsäurechlorid umgewandelt werden. Diese Verbindung kann beispielsweise im Rahmen der Herstellung von Pharmazeutika und Agrochemikalien verwendet werden.

Die benötigten Ausgangsmaterialien sind bekannt und nach Standardmethoden herstellbar. Die Herstellung von 1.1.1.2.2.-Pentafluoro-3.3-dichloropropan aus 1.1.1.2.2.-Pentafluoro-3.3.3-trichloropropan und Wasserstoff über Iridiumkatalysatoren wird beispielsweise in der japanischen Offenlegungsschrift 04/210 653 beschrieben.

Eine besonders bevorzugte Ausführungsform betrifft die Herstellung von Trifluoracetylchlorid und Chlordifluoracetylchlorid.

Diese bevorzugte Ausführungsform umfaßt die Herstellung von Verbindungen der allgemeinen Formel $CF_2XC(O)Cl$, worin X = Cl, F, also von Trifluoracetylchlorid und Chlordifluoracetylchlorid und ist dadurch gekennzeichnet, daß man 1,1-Difluor-1,2,2-trichlorethan (R122) bzw. 1,1,1-Trifluor-2,2-dichlorethan mit Sauerstoff in der Gasphase kontinuierlich unter aktivierender Bestrahlung ohne Zusatz von elementarem Chlor drucklos umsetzt. Anhand dieser Ausführungsform wird die Erfindung weiter erläutert.

Die aktivierende Bestrahlung wird vorzugsweise mit Strahlern durchgeführt, die Licht abgeben, das mindestens teilweise im UV-Bereich liegt. Beispielsweise sind Hg-Hochdruck-, Mitteldruck- und Niederdruckstrahler geeignet. Als Werkstoff für die entsprechenden Apparaturbestandteile empfiehlt sich UV-durchlässiges Material, beispielsweise Quarzglas, sofern man von außen bestrahlt.

Die Umsetzung wird vorzugsweise bei einer Temperatur von bis zu 200 °C, insbesondere in einem Temperaturbereich von 80 bis 130 °C durchgeführt. Das Mol-Verhältnis zwischen der Ausgangsverbindung und Sauerstoff ($O_2$)

liegt vorzugsweise im Bereich von 1:1 bis 1:20, insbesondere 1:1,1 bis 1:3. Sauerstoff kann als sauerstoffhaltiges Gas, z.B. in Form von Luft, vorzugsweise als reiner Sauerstoff eingesetzt werden.

Zum Schutz der verwendeten Apparatur soll die Kondensation von Reaktanten in der Apparatur nach Möglichkeit vermieden werden. Auch ist es in bezug auf die Produktreinheit wünschenswert, daß möglichst wenig Wasser bei der Umsetzung vorhanden ist. Gewünschtenfalls kann man die Reaktanten in bekannter Weise von mitgeschlepptem Wasser befreien, beispielsweise durch Kontaktieren mit Trocknungsmitteln wie Trockenperlen oder Phosphorpentoxid.

In kontinuierlicher Verfahrensweise wird gasförmiges 1,1-Difluor-1,2,2-trichlorethan bzw. 1,1,1-Trifluor-2,2-dichlorethan und Sauerstoff dem Reaktionsgefäß zugeführt und aus dem Reaktionsgefäß Produkt kontinuierlich abgeführt. Natürlich werden die Mengen an zugeführten Ausgangsverbindungen und abgeführtem Produkt aufeinander abgestimmt.

Die durchschnittliche Verweilzeit im Reaktionsgefäß liegt vorzugsweise zwischen 0,1 und 30 min. Die optimale durchschnittliche Verweilzeit, die u. a. von der Lampenleistung und von geometrischen Parametern der Bestrahlungsapparatur abhängig ist, kann man durch einfache Handversuche und Analyse des Produktstroms, z. B. durch Gaschromatographie, ermitteln.

Es hat sich gezeigt, daß bessere Umsatzraten und insbesondere höhere Selektivität erzielt werden können, wenn statt einer einzelnen Bestrahlungslampe mit bestimmter Leistung zwei oder mehr leistungsschwächere Lampen gleicher Gesamtleistung in hintereinandergeschalteten Reaktoren eingesetzt werden. Auch ist eine gute Verwirbelung der Reaktionsmischung, z. B. durch geeignete Einbauten im Reaktor, von Vorteil.

Ein Vorteil des erfindungsgemäßen Verfahrens besteht darin, daß Polyfluor- und- Perfluoracetylchloride mit hoher Selektivität hergestellt werden können.

Das erfindungsgemäße Verfahren gestattet gemäß einer Variante die Herstellung von Chlordifluoracetylchlorid aus 1,1-Difluor-1,2,2-trichlorethan ohne Chlorzusatz unter Bestrahlung mit Sauerstoff in sehr hoher Ausbeute. Dieses Resultat muß als überraschend angesehen werden. Zwar wird die Oxidation von Polyhalogeno-Verbindungen mit Sauerstoff unter aktivierender Bestrahlung schon von den Autoren W. C. Francis und R. N. Haszeldine in J. Chem. Soc. 1955, Seiten 2151-2163 und von R. N. Haszeldine und F. Nyman in J. Chem. Soc. 1959, Seiten 387-396 beschrieben. Bei den von den genannten Autoren beschriebenen Versuchen, die stets bei erhöhtem Druck durchgeführt wurden, ergab sich aber, daß Verbindungen mit der $CF_2Cl$-Gruppe unter Bildung von Kohlendioxid und Siliciumtetrafluorid (Reaktion mit dem Glasmaterial des Reaktionsgefäßes) zerfallen. Die hohe Selektivität und hohe Reaktionsgeschwindigkeit der photochemischen Oxidation von R122 ohne Chlorzusatz war deshalb nicht vorhersehbar. Auch die bei der anderen Variante der Erfindung, nämlich der Herstellung von Trifluoracetylchlorid, drucklos durchgeführt, resultierende hohe Ausbeute muß als unerwartet bezeichnet werden. Bislang herrschte nämlich in der Fachwelt die Ansicht, diese Umsetzung müsse unter Chlorzusatz bei erhöhtem Druck (bzw. dem der erhöhten Temperatur entsprechenden Überdruck) durchgeführt werden. Erstaunlicherweise werden gemäß dem Verfahren der Erfindung auch Glasapparaturen nicht angegriffen. Vorteil des erfindungsgemäßen Verfahrens ist, daß keine unerwünschten Nebenprodukte entstehen, die ansonsten bei Chlorzusatz gebildet werden.

Wie festgestellt wurde, weisen Chlorfluorkohlenwasserstoffe bei erhöhtem Druck in Anwesenheit von Sauerstoff möglicherweise Bereiche erhöhter Explosionsgefahr auf. Das erfindungsgemäße Verfahren gestattet demgegenüber eine gefahrlose Herstellung der Säurehalogenide.

Die folgenden Beispiele sollen die Erfindung weiter erläutern, ohne sie in ihrem Umfang einzuschränken.

**<u>Beispiel 1:</u>**

Kontinuierliche Herstellung von Chlordifluoracetylchlorid durch photochemische Oxidation von $CF_2Cl\text{-}CHCl_2$ (122) mit Sauerstoff

$$CF_2Cl\text{-}CHCl_2 + O_2 \xrightarrow{h\nu} CF_2ClC(O)Cl + HCl + \tfrac{1}{2}\,O_2$$

In einem 400 ml-Photolyse-Tauchschachtreaktor wurde bei 100 °C Reaktorinnentemperatur eine Mischung aus $CF_2Cl\text{-}CHCl_2$ (aus Vorverdampfer mit T = 150 °C) und reinem Sauerstoff im Molverhältnis 1:2,2 unter Ausschluß von Feuchtigkeit und ohne Chlorzusatz gasförmig zudosiert und währenddessen mit einem Hg-Hochdruckstrahler TQ 718 der Firma Heraeus (700 W) durch Quarzglas bestrahlt. Die Kondensation der gasförmig den Reaktor verlassenden Produkte erfolgte in einem nachgeschalteten auf -60 °C temperierten Kühler (HCl- und $COF_2$-Austrieb). Umgesetzt wurden innerhalb von 30 min. 155,9 g (920 mmol) $CF_2ClCHCl_2$. Der Umsatz betrug 89 %. Die Ausbeute an Chlordifluoracetylchlorid betrug 92,9 % d. Theorie (GC-Prozent) neben $COCl_2$, $CO_2$ und $COF_2$ als Nebenkomponenten. Eine

anschließende Feindestillation über eine 40 cm Füllkörperkolonne ergab nach Abnahme eines $COCl_2$-Vorlaufes reines Chlordifluoracetylchlorid (Kp. 27 °C). Zurückgewonnenes R122 wurde mit einer Pumpe erneut in den Vorverdampfer eindosiert.

**Beispiel 2:**

Kontinuierliche Herstellung von Trifluoracetylchlorid durch photochemische Oxidation von $CF_3$-$CHCl_2$ (123) mit Sauerstoff

$$CF_3\text{-}CHCl_2 + O_2 \xrightarrow{h\nu} CF_3C(O)Cl + HCl + ½\ O_2$$

In einem 400 ml Photolyse-Tauchschachtreaktor wurde bei 100 °C Reaktorinnentemperatur eine Mischung aus $CF_3$-$CHCl_2$ (aus Vorverdampfer mit T = 100 °C) und reinem Sauerstoff im Molverhältnis 1:1,4 gasförmig ohne Chlorzusatz zudosiert und währenddessen mit einem Hg-Hochdruckstrahler TQ 718 von Heraeus (Einstellung auf 500 W Leistung) durch Quarzglas bestrahlt. Die Kondensation der gasförmig den Reaktor verlassenden Produkte erfolgte in einem nachgeschalteten auf -60 °C temperierten Kühler (HCl und $COF_2$-Austrieb). Umgesetzt wurden innerhalb von 30 min 0,96 mol $CF_3CHCl_2$. Die Ausbeute an Trifluoracetylchlorid (70 % 123-Umsatz) betrug 93,2 % d. Theorie (GC-Prozent). Als Nebenkomponenten im Reaktorgas wurden $COCl_2$, $CO_2$ und $COF_2$ nachgewiesen. Eine anschließende Feindestillation des Kondensates in einer Füllkörperkolonne mit Intensivkühlung ergab reines Trifluoracetylchlorid (Kp. -24,8 °C).

**Beispiel 3:**

Durchführung mit erhöhter Lampenleistung

Durchführung wie Beispiel 2, jedoch mit einer Lampenleistung von 700 W. Der Umsatz an 123 lag hier bei 93 %, die Ausbeute an Trifluoracetylchlorid bei 84 % (Nebenkomponenten und Aufarbeitung wie in Beispiel 2) (GC-Prozent).

**Beispiel 4:**

Durchführung mit verringertem Verhältnis R123:$O_2$

Durchführung wie Beispiel 3, jedoch mit einem 123/$O_2$-Verhältnis von 1:0,6. Der Umsatz an 123 lag bei 58 %, die Ausbeute an Trifluoracetylchlorid bei 89 % (GC-Prozent).

**Beispiel 5:**

Durchführung mit erhöhtem Verhältnis R123:$O_2$

Durchführung wie Beispiel 3, jedoch mit einem 123/$O_2$-Verhältnis von 1:2,28. Der Umsatz an 123 lag bei 95 %, die Ausbeute an Trifluoracetylchlorid bei 85 % (GC-Prozent).

**Beispiel 6:**

Herstellung von Perfluorpropionylchlorid

$$CF_3CF_2CHCl_2 + 1/2\ O_2 \rightarrow CF_3CF_2C(O)Cl$$

Analog zu Beispiel 1 wurde eine Mischung aus 225 ca (98 % Reinheit; aus Vorverdampfer mit T = 130 °C) und reinem Sauerstoff im Molverhältnis 1 : 1,7 gasförmig zudosiert und währenddessen durch Quarzglas bestrahlt (Lampeneinstellung: 500 W). Die Dosierung an 225 ca betrug 0,45 mol/30 min. Die Selektivität bezüglich Perfluorpropionylchlorid betrug 68 %, der Umsatz lag bei 63 %.

## Patentansprüche

1. Verfahren zur Herstellung von Verbindungen der allgemeinen Formel RCFXC(O)Cl, worin X für Fluor oder Chlor steht und R für Fluor oder einen perfluorierten C1-C10-Alkylrest, durch Umsetzung von $RCFXCHCl_2$, worin R und X die oben genannte Bedeutung besitzen, mit Sauerstoff in der Gasphase unter aktivierender Bestrahlung, mit der Maßgabe, daß man die Umsetzung kontinuierlich ohne Zusatz von elementarem Chlor drucklos durchführt.

2. Verfahren nach Anspruch 1 zur Herstellung von Verbindungen der Formel $CF_2XC(O)Cl$, worin X = Cl,F bedeutet, also von Trifluoracetylchlorid und von Chlordifluoracetylchlorid, dadurch gekennzeichnet, daß man 1,1-Difluor-1,2,2-tri-chlorethan (R122) bzw. 1,1,1-Trifluor-2,2-dichlorethan (R123) einsetzt.

3. Verfahren nach einem der vorhergehenden Ansprüche, dadurch gekennzeichnet, daß man die Umsetzung bei einer Temperatur von bis zu 200 °C, vorzugsweise in einem Temperaturbereich von 80 bis 130 °C durchführt.

4. Verfahren nach einem der vorhergehenden Ansprüche, dadurch gekennzeichnet, daß man das Verfahren kontinuierlich durchführt, indem man das gasförmige Ausgangsmaterial, vorzugsweise gasförmiges 1,1-Difluor-1,2,2-trichlorethan bzw. 1,1,1-Trifluor-2,2-dichlorethan, und Sauerstoff einem Reaktionsgefäß zuführt und aus dem Reaktionsgefäß das produkt kontinuierlich abführt, wobei die durchschnittliche Verweilzeit im Reaktionsgefäß zwischen 0,1 und 30 min. liegt.

5. Verfahren nach einem der vorhergehenden Ansprüche, dadurch gekennzeichnet, daß das Mol-Verhältnis zwischen der Ausgangsverbindung und Sauerstoff ($O_2$) im Bereich von 1:1 bis 1:20 liegt.

## Claims

1. Process for the preparation of compounds of the general formula RCFXC(O)Cl, in which X is fluorine or chlorine and R is fluorine or a perfluorinated C1-C10-alkyl radical, by reaction of $RCFXCHCl_2$, in which R and X have the above meaning, with oxygen in the gas phase under activating irradiation, with the proviso that the reaction is carried out continuously without addition of elemental chlorine and unpressurized.

2. Process according to Claim 1 for the preparation of compounds of the formula $CF_2XC(O)Cl$, in which X is Cl, F, hence of trifluoroacetyl chloride and of chlorodifluoroacetyl chloride, characterized in that 1,1-difluoro-1,2,2-trichloroethane (R122) or 1,1,1-trifluoro-2,2-dichloroethane (R123) is used.

3. Process according to one of the preceding claims, characterized in that the reaction is carried out at a temperature of up to 200°C, preferably in a temperature range from 80 to 130°C.

4. Process according to one of the preceding claims, characterized in that the process is carried out continuously by feeding the gaseous starting material, preferably gaseous 1,1-difluoro-1,2,2-trichloroethane or 1,1,1-trifluoro-2,2-dichloroethane, and oxygen into a reaction vessel and continuously taking the product from the reaction vessel, with the average residence time in the reaction vessel being between 0.1 and 30 minutes.

5. Process according to one of the preceding claims, characterized in that the molar ratio between the starting compound and oxygen ($O_2$) is in the range from 1:1 to 1:20.

## Revendications

1. Procédé de préparation de composés de formule générale RCFXC(O)Cl, dans laquelle X représente un fluor ou un chlore et R représente un fluor ou un radical alkyle en $C_1$ à $C_{10}$ perfluoré, par réaction de $RCFXCHCl_2$, où R et X ont la signification donnée ci-dessus, avec de l'oxygène en phase gazeuse sous une irradiation activante, sous réserve que l'on conduise la réaction sans pression de façon continue sans addition de chlore élémentaire.

2. Procédé selon la revendication 1 pour la préparation de composés de formule $CF_2XC(O)Cl$, dans laquelle X = Cl, F, donc de chlorure de trifluoroacétyle et de chlorure de chlorodifluoroacétyle, caractérisé en ce qu'on utilise le 1,1-difluoro-1,2,2-trichloroéthane ($R_{122}$) ou selon les cas le 1,1,1-trifluoro-2,2-dichloroéthane ($R_{123}$).

3. Procédé selon l'une des revendications précédentes, caractérisé en ce qu'on conduit la réaction à une température allant jusqu'à 200°C, de préférence dans un intervalle de température allant de 80 à 130°C.

4. Procédé selon l'une des revendications précédentes, caractérisé en ce qu'on conduit le procédé de façon continue en introduisant dans un vase à réaction le produit de départ gazeux, de préférence le 1,1-difluoro-1,2,2-trichloroéthane, ou selon les cas, le 1,1,1-trifluoro-2,2-dichloroéthane gazeux, et de l'oxygène, et en ce qu'on retire le produit de façon continue du vase à réaction, le temps de séjour moyen dans le vase à réaction étant compris entre 0,1 et 30 minutes.

5. Procédé selon l'une des revendications précédentes, caractérisé en ce que le rapport molaire entre le composé de départ et l'oxygène ($O_2$) est compris dans un intervalle de 1:1 à 1:20.